# EUROPEAN PATENT APPLICATION

(11) **EP 4 729 513 A1**
(43) Date of publication of application: **22.04.2026**
(21) Application number: 24830335.6
(22) Date of filing: 27.05.2024
(51) Int. Cl.: C07D 277/46, A61K 8/49, A61Q 19/02, A61Q 19/00, A61K 31/426, A61K 31/427, A61K 31/5377, A61K 31/541, A61P 17/00

(54) **A-KETO ACID AMIDE OR SUBSTITUTED OXALIC ACID AMIDE ESTER COMPOUND AND COMPOSITION THEREOF**

(30) Priority: 29.06.2023 CN 202310777411
(71) Applicant: Nanjing Chempion Biotechnology Co., Ltd., Nanjing, Jiangsu 210000 (CN); PROYA COSMETICS CO., LTD., Zhejiang 311000 (CN)
(72) Inventor: CHEN, Jian, Nanjing, Jiangsu 210000 (CN); YU, Zhangquan, Nanjing, Jiangsu 210000 (CN); CHEN, Tao, Nanjing, Jiangsu 210000 (CN)
(74) Representative: Bayramoglu et al.
(86) International application number: PCT/CN2024/095545
(87) International publication number: WO 2025/001687

(57) **Abstract**

The present disclosure relates to the technical field of chemical synthesis, and particularly relates to an α-ketoamide or substituted oxamide ester compound and a composition including the same. The α-ketoamide or substituted oxamide ester compound has a general structural formula shown in formula I: where R₁ is C₁-C₆ alkyl or cycloalkyl, or OR₂, where R₂ is C₁-C₈ alkyl or cycloalkyl, or NR₃R₄, where R₃ and R₄ are each independently C₁-C₆ alkyl or cycloalkyl; or is an amine group with the following structure: where X and Y are each independently C₁-C₅ methylene and Z is one of CH₂, O, S, and NH. The α-ketoamide or substituted oxamide ester compound of the present disclosure can effectively inhibit tyrosinase (TYR) activity to suppress melanin synthesis, and exhibits an excellent skin-whitening effect. Compared with the prior art, the molecular spatial structure of the α-ketoamide or substituted oxamide ester compound in the present disclosure is significantly altered, and possesses increased interaction sites with TYR and improved solubility, resulting in superior efficacy.

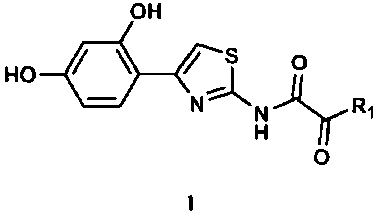

## Description

### TECHNICAL FIELD

The present disclosure relates to the technical field of chemical synthesis, and particularly relates to an α-ketoamide or substituted oxamide ester compound and a composition including the same.

### BACKGROUND TECHNOLOGY

Skin pigmentation, typically such as melasma, freckles, and postinflammatory hyperpigmentation, is a major cosmetic concern for which many patients seek medical attention. Melanin in human epidermal cells is the primary endogenous cause of skin pigmentation. Under normal physiological conditions, the production of melanin protects the skin from ultraviolet damage and helps prevent DNA damage caused by light radiation. However, excess melanin can lead to skin pigmentation.

Melanin production is a complex process involving a combination of enzymatic and chemical catalytic reactions. Melanin is primarily synthesized and secreted by melanocytes. Each melanocyte is associated with surrounding keratinocytes to form a dendritic structure. Melanosomes are specialized organelles within melanocytes, and include melanin granules. The synthesis, storage, and transport of melanin granules all occur within melanosomes. After being generated, melanin is transferred from dendrite tips of melanocytes to keratinocytes. Melanocytes produce two types of melanin: eumelanin and pheomelanin. The melanin synthesis process begins with the oxidation of tyrosine into dopaquinone by tyrosinase (TYR). The generation of dopaquinone is a rate-limiting step in melanin synthesis. After being generated, dopaquinone can undergo further conversion in the presence of cysteine or glutathione to ultimately produce pheomelanin. Alternatively, the generated dopaquinone may undergo intramolecular cyclization to produce cyclodopa, cyclodopa is then further oxidized to generate dopachrome, and dopachrome is eventually converted into eumelanin under the action of tyrosinase-related proteins (TRPs) such as TRP-1 and TRP-2. Although TYR, TRP-1, and TRP-2 are all involved in the melanin production pathway, TYR is the sole essential enzyme for melanin production. Consequently, inhibiting the activity of TYR can directly affect the rate and quantity of melanin synthesis, thereby reducing hyperpigmentation. This strategy is currently the primary focus of research for whitening agents on the market.

Currently, many topical products are available for treating pigmentation disorders. These topical products include a variety of active ingredients to reduce the production and/or distribution of melanin. Common active ingredients include hydroquinone, kojic acid, and arbutin, among others. However, the existing common treatment solutions often have safety risks that cannot be overlooked. For example, hydroquinone may cause exogenous ochronosis and permanent leukoderma. Arbutin, as a derivative of hydroquinone, carries a non-negligible risk of releasing hydroquinone under some conditions. The use of kojic acid has remained controversial due to potential endocrine-disrupting properties of kojic acid. In 2013, the Japanese cosmetics company Kanebo incorporated rhododenol into products to reduce melanin production. However, rhododenol-containing products led to severe side effects, including symptoms of leukoderma. Thus, these rhododenol-containing products were eventually recalled and withdrawn from the market. A significant number of affected users have still not recovered.

Many compounds with resorcinol structural units also possess the ability to inhibit TYR and reduce melanin production, and thus are utilized in various commercial whitening products, such as isobutyrylaminothiazolyl resorcinol, dimethoxytolyl-4-propylresorcinol, phenylethyl resorcinol, butyl resorcinol, and hexyl resorcinol. This class of compounds generally exhibit prominent *in vitro* TYR inhibitory activity, but often suffer from drawbacks such as poor water solubility, susceptibility to degradation/discoloration, generation of unpleasant odors, and strong irritability.

Therefore, there is an urgent need to develop novel, safe, efficient, and user-friendly whitening products.

### CONTENT OF THE INVENTION

An objective of the present disclosure is to overcome the deficiencies in the prior art and provide an α-ketoamide or substituted oxamide ester compound capable of effectively inhibiting TYR activity and suppressing melanin synthesis.

To achieve the above objective, the present disclosure adopts the following technical solutions:
The present disclosure provides an α-ketoamide or substituted oxamide ester compound with a general structural formula shown in formula I: 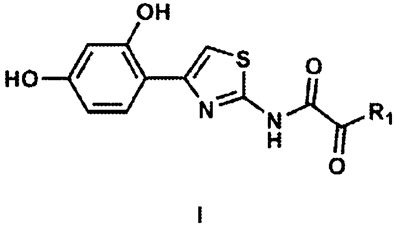
where R₁ is C₁-C₆ alkyl or cycloalkyl, or OR₂, where R₂ is C₁-C₈ alkyl or cycloalkyl, or NR₃R₄, where R₃ and R₄ are each independently C₁-C₆ alkyl or cycloalkyl; or
is an amine group with the following structure: 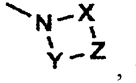 where X and Y are each independently C₁-C₅ methylene and Z is one of CH₂, O, S, and NH.

Further, the α-ketoamide or substituted oxamide ester compound exists in a free form or as a pharmaceutically acceptable salt or ester thereof.

The present disclosure also provides a composition including the α-ketoamide or substituted oxamide ester compound. The composition is a cosmetic or dermatological preparation.

Further, an addition amount of the α-ketoamide or substituted oxamide ester compound in the composition ranges from 0.000001% to 10% of a total weight of a preparation.

The technical solutions adopted in the present disclosure have the following beneficial effects:
(1) The α-ketoamide or substituted oxamide ester compound of the present disclosure can effectively inhibit TYR activity to suppress melanin synthesis, and exhibits an excellent skin-whitening effect.
(2) Compared with the prior art, due to technical improvements, the molecular spatial structure of the α-ketoamide or substituted oxamide ester compound in the present disclosure is significantly altered, and possesses increased interaction sites with TYR and improved solubility, resulting in superior efficacy.

### SPECIFIC IMPLEMENTATIONS

The present disclosure is further described below through examples, but the present disclosure is not limited to the scope of the examples. The experimental methods which are not specified with specific conditions in the following examples are conducted according to conventional conditions or according to product instructions.

4-(2,4-Dimethoxyphenyl)-1,3-thiazol-2-amine can be conveniently prepared by a published method (reference: US2011/0230486) or can be directly obtained as a commercially available reagent.

### Example 1:

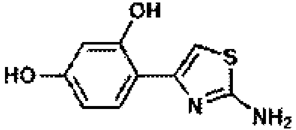

Under nitrogen protection, 4-(2,4-dimethoxyphenyl)-1,3-thiazol-2-amine (1.5 g) was dispersed in dry dichloromethane (30 mL), and cooling was conducted to -5°C to 10°C. Then, a solution of boron tribromide in dichloromethane (2.0 M, 19 mL) was added dropwise, and a reaction was conducted at a specified temperature for 24 h. After the reaction was completed, methanol was added dropwise for quenching. The resulting reaction solution was concentrated and dispersed in ethyl acetate (100 mL) for extraction. The resulting organic phase was washed with saturated sodium bicarbonate and saturated brine, and then dried over anhydrous sodium sulfate. A dried organic phase was concentrated, filtered, washed with a small amount of cold isopropyl acetate, and vacuum-dried at 20°C to 30°C to produce 0.69 g of 4-(2-aminothiazol-4-yl)benzene-1,3-diol as a brown solid, which could be used directly in a subsequent reaction without purification.

### Example 2:

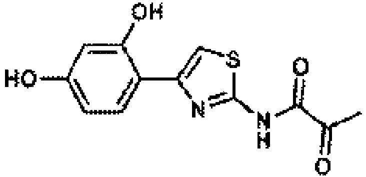

4-(2-Aminothiazol-4-yl)benzene-1,3-diol (0.50 g) was dissolved in anhydrous tetrahydrofuran (THF) (10 mL). At 0°C to 10°C, a solution of 2-oxopropanoyl chloride (which could be prepared by a method in Org. Synth. 1983, 61, 1; 0.42 g) in anhydrous THF (1.6 mL) was added dropwise. After the dropwise addition was completed, a reaction was conducted overnight at a specified temperature. Subsequently, methylamine (33% ethanol solution, 3 mL) was added, and stirring was conducted for 2 h. Vacuum concentration was conducted to remove the solvents. The resulting mixture was acidified with 1 N HCl. Recrystallization was conducted with isopropanol-water to produce 0.28 g of N-(4-(2,4-dihydroxyphenyl)thiazol-2-yl)-2-oxopropanamide as an off-white solid. ESI-MS: m/z301.1 [M+Na]⁺; ¹H NMR(400MHz,DMSO-d₆): 12.05(s,1H),10.97(s,1H), 9.71(s,1H),7.57(m,1H), 7.43(s,1H),6.44(m,1H),6.35(m,1H),2.15(s,3H).

### Example 3:

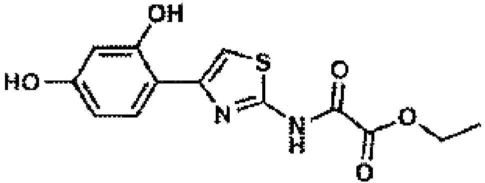

4-(2-Aminothiazol-4-yl)benzene-1,3-diol (0.43 g) was dissolved in anhydrous THF (8 mL). At 0°C to 10°C, a solution of ethyl chloroglyoxylate (0.50 g) in anhydrous THF (2 mL) was added dropwise. After the dropwise addition was completed, a reaction was conducted overnight at a specified temperature. Subsequently, methylamine (33% ethanol solution, 2.5 mL) was added, and stirring was conducted for 2 h. Vacuum concentration was conducted to remove the solvents. The resulting mixture was separated by column chromatography (silica gel, dichloromethane-methanol) to produce 0.38 g of ethyl 2-((4-(2,4-dihydroxyphenyl)thiazol-2-yl)amino)-2-oxoacetate as a white solid. ESI-MS: m/z309.0[M+H]⁺, 331.0[M+Na]⁺; ¹H NMR (400MHz, DMSO- d₆) :12.13(s,1H),10.20(s,1H),9.64(s,1H), 7.55(m,1H),7.44(s,1H),6.50(m,1H),6.44 (m,1H) ,4.30(s,2H),1.30(t,3H).

### Example 4:

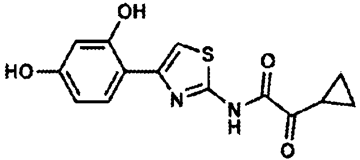

2-Cyclopropyl-2-oxoacetic acid (0.21 g) was dispersed in dry acetonitrile (15 mL). *N,N-*diisopropylethylamine (DIPEA) (0.55 g) and 1-[bis(dimethylamino)methylene]-1*H*-1,2,3-triazolo[4,5-b]pyridinium 3-oxide hexafluorophosphate (HATU) (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by column chromatography (silica gel, dichloromethane-methanol) to produce 0.14 g of 2-cyclopropyl-N-(4-(2,4-dihydroxyphenyl)thiazol-2-yl)-2-oxoacetamide as an off-white solid. ESI-MS: m/z327.1[M+Na]⁺;¹H NMR(400MHz,DMSO-d₆): 12.01(s,1H),10.10(s,1H),9.71(s,1H),7.53(m,1H), 7.40(s,1H),6.45(m,1H), 6.34(m,1H),1.81(m,1H),0.97(m,2H),0.75(m,2H).

### Example 5:

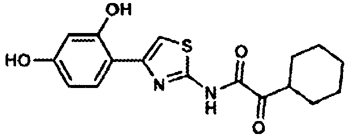

2-Cyclohexyl-2-oxoacetic acid (0.28 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by column chromatography (silica gel, dichloromethane-methanol) to produce 0.23 g of 2-cyclohexyl-N-(4-(2,4-dihydroxyphenyl)thiazol-2-yl)-2-oxoacetamide as a white solid. ESI-MS: m/z369.1[M+Na]⁺; ¹H NMR (400MHz,DMSO-d₆):12.09(s,1H),10.13(s,1H),9.64(s,1H),7.60(m,1H),7.43(s,1H),6.42(m,1H),6.33(m,1H),2.31(m,1 H)1.80(m,2H),1.62-1.37(m,8H).

### Example 6:

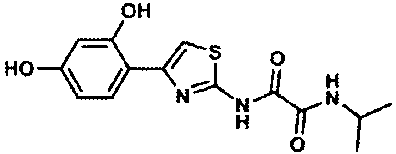

2-Oxo-2-(prop-2-ylamino)acetic acid (0.24 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by preparative high-performance liquid chromatography (prep HPLC) (ACCQPrep HP150, Welch Ultimate Polar-RP 5 µm 21.2 × 150 mm, (A) 0.1% TFA aq., and (B) CH₃CN). A purified product was collected and lyophilized to produce 0.16 g of *N*-(4-(2,4-dihydroxyphenyl)thiazol-2-yl)-*N*'-isopropyloxalamide as a white powder. ESI-MS: m/z322.1[M+H]⁺, 344.1[M+Na]⁺; ¹H NMR(400MHz,DMSO-d₆):11.99(s,1H),10.23(s,1H), 9.68(s,1H),7.97(s,1H),7.45(m,1H),7.33(s,1H),6.38-6.32(m,2H),4.07(m,1H),1.24(d,6H).

### Example 7:

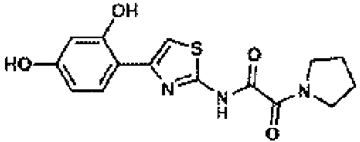

Oxo(1-pyrrolidinyl)acetic acid (0.26 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by prep HPLC (ACCQPrep HP150, Welch Ultimate Polar-RP 5 µm 21.2 × 150 mm, (A) 0.1% TFA aq., and (B) CH₃CN). A purified product was collected and lyophilized to produce 0.25 g of *N*-(4-(2,4-dihydroxyphenyl)thiazol-2-yl)-*N*"-isopropyloxalamide as a white powder. ESI-MS: m/z334.1[M+H]⁺, 356.1 [M+Na]⁺; ¹HNMR(400MHz,DMSO-d₆):12.13(s,1H),10.30(s,1H), 9.86(s,1H), 7.53(s,1H), 7.45(s,1H),6.51(m,1H),6.43(m,1H),3.42(m,4H),1.81(m,4H).

### Example 8:

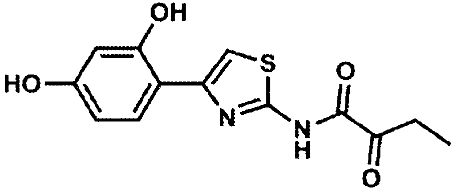

4-(2-Aminothiazol-4-yl)benzene-1,3-diol (0.50 g) was dissolved in anhydrous THF (10 mL). At 0°C to 10°C, a solution of 2-oxobutanoyl chloride (0.49 g) in anhydrous THF (1.6 mL) was added dropwise. After the dropwise addition was completed, a reaction was conducted overnight at a specified temperature. Subsequently, methylamine (33% ethanol solution, 3 mL) was added, and stirring was conducted for 2 h. Vacuum concentration was conducted to remove the solvents. The resulting mixture was acidified with 1 N HCl. Recrystallization was conducted with isopropanol-water to produce 0.22 g of N-(4-(2,4-dihydroxyphenyl)thiazol-2-yl)-2-oxobutanamide as an off-white solid. ESI-MS: m/z315.1[M+Na]⁺; ¹H NMR(400MHz,DMSO-d₆):12.25(s,1H),11.03 (s,1H),9.72(s,1H),7.60(m,1H),7.43(s,1H),6.44(m,1H),6.35(m,1H),2.39(q,J=7.2Hz,2H), 1.06(t,J=7.2Hz,2H).

### Example 9:

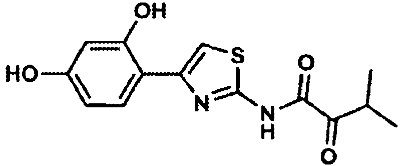

4-(2-Aminothiazol-4-yl)benzene-1,3-diol (0.50 g) was dissolved in anhydrous THF (10 mL). At 0°C to 10°C, a solution of 3-methyl-2-oxobutanoyl chloride (0.55 g) in anhydrous THF (1.6 mL) was added dropwise. After the dropwise addition was completed, a reaction was conducted overnight at a specified temperature. Subsequently, methylamine (33% ethanol solution, 3 mL) was added, and stirring was conducted for 2 h. Vacuum concentration was conducted to remove the solvents. The resulting mixture was separated by column chromatography (dichloromethane-methanol) to produce 0.18 g of N-(4-(2,4-dihydroxyphenyl)thiazol-2-yl)-3-methyl-2-oxobutanamide. ESI-MS: m/z 328.9[M+Na]⁺; ¹H NMR(400MHz,DMSO-d₆):12.11(s,1H),11.07 (s,1H),9.71(s,1H),7.62(m,1H),7.43(s,1H),6.42(m,1H),6.34(m,1H),3.11(m,1H),1.04(d,J=6.9Hz,6 H).

### Example 10:

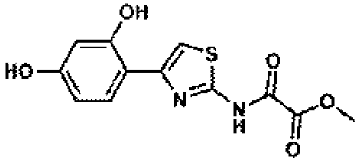

4-(2-Aminothiazol-4-yl)benzene-1,3-diol (0.43 g) was dissolved in anhydrous THF (8 mL). At 0°C to 10°C, a solution of methyl chloroglyoxylate (0.47 g) in anhydrous THF (2 mL) was added dropwise. After the dropwise addition was completed, a reaction was conducted overnight at a specified temperature. Subsequently, methylamine (33% ethanol solution, 2.5 mL) was added, and stirring was conducted for 2 h. Vacuum concentration was conducted to remove the solvents. The resulting mixture was separated by column chromatography (silica gel, dichloromethane-methanol) to produce 0.32 g of methyl 2-((4-(2,4-dihydroxyphenyl)thiazol-2-yl)amino)-2-oxoacetate. ESI-MS: m/z294.9[M+H]⁺, 316.9[M+Na]⁺; ¹H NMR(400MHz,DMSO-d₆):12.07(s,1H),10.33(s,1H),9.67(s,1H),7.61(m,1H),7.46(s,1H),6.45(m,1H),6.36(m,1H),3.58(s,3 H).

### Example 11:

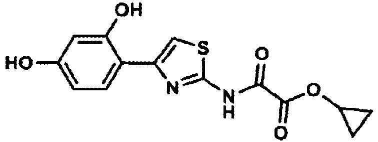

2-Cyclopropoxy-2-oxoacetic acid (0.24 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by prep HPLC (ACCQPrep HP150, Welch Ultimate Polar-RP 5 µm 21.2 × 150 mm, (A) 0.1% TFA aq., and (B) CH₃CN). A purified product was collected and lyophilized to produce 0.22 g of cyclopropyl 2-((4-(2,4-dihydroxyphenyl)thiazol-2-yl)amino)-2-oxoacetate. ESI-MS: m/z 342.9 [ M+Na]⁺ ; ¹H NMR(400MHz,DMSO-d₆):12.12(s,1H),10.24(s,1H),9.68(s,1H), 7.63(m,1H),7.51(s,1H), 6.47(m,1H),6.35(m,1H),0.66-0.25(m,4H).

### Example 12:

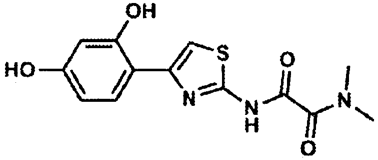

*N*,*N*-Dimethyloxamic acid (0.21 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by prep HPLC (ACCQPrep HP150, Welch Ultimate Polar-RP 5 µm 21.2 × 150 mm, (A) 0.1% TFA aq., and (B) CH₃CN). A purified product was collected and lyophilized to produce 0.28 g of *N*-(4-(2,4-dihydroxyphenyl)thiazol-2-yl)-*N'*,*N'-*dimethyloxalamide. ESI-MS: m/z308.1[M+Ht, 330.1[M+Na]⁺; ¹H NMR(400MHz,DMSO-d₆):12.11(s,1H),10.27(s,1H),9.87(s,1H),7.58(s,1H), 7.42(s,IH),6.46 (m,1H),6.33(m,1H),2.98(s,6H).

### Example 13:

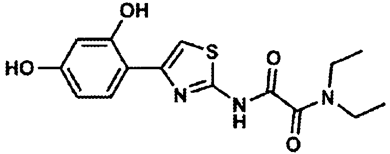

*N*,*N*-Diethyloxamic acid (0.32 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by prep HPLC (ACCQPrep HP150, Welch Ultimate Polar-RP 5 µm 21.2 × 150 mm, (A) 0.1% TFA aq., and (B) CH₃CN). A purified product was collected and lyophilized to produce 0.31 g of *N*-(4-(2,4-dihydroxyphenyl)thiazol-2-yl)-*N*',*N*'-diethyloxalamide. ESI-MS: m/z358.1[M+Na]⁺; ¹H NMR(400MHz, DMSO-d₆):12.34(s,1H),10.31(s,1H),9.89(s,1H),7.56(s,1H),7.45(s,1H),6.44(m,1H),6.35 (m,1H),3.05(q,J=7.1Hz,4H),1.15(t,J=7.1Hz,6H).

### Example 14:

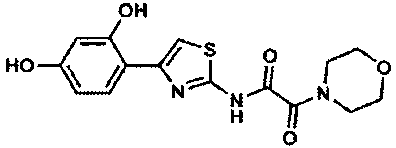

2-Morpholino-4-yl-2-oxoacetic acid (0.35 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by prep HPLC (ACCQPrep HP150, Welch Ultimate Polar-RP 5 µm 21.2 × 150 mm, (A) 0.1% TFA aq., and (B) CH₃CN). A purified product was collected and lyophilized to produce 0.29 g of *N*-(4-(2,4-dihydroxyphenyl)thiazol-2-yl)-2-morpholino-2-oxoacetamide. ESI-MS: m/z372.1[M+Na]⁺; ¹H NMR(400MHz,DMSO-d₆):12.22(s,1H),10.27(s,1H),9.76(s,1H),7.55(s,1H), 7.37 (s,1H),6.42(m,1H),6.31(m,1H),3.65(d,4H),3.44(d,4H).

### Example 15:

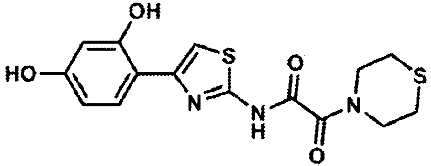

Oxo(thiomorpholin-4-yl)acetic acid (0.38 g) was dispersed in dry acetonitrile (15 mL). DIPEA (0.55 g) and HATU (0.72 g) were then added, and stirring was conducted at room temperature for 30 min. 4-(2-Aminothiazol-4-yl)benzene-1,3-diol (0.30 g) was added, and stirring was conducted to allow a reaction for 12 h to 24 h. After the reaction was completed, 1 N NaOH (15 mL) was added, and stirring was conducted overnight at 5°C to 15°C. The resulting reddish-brown mixed solution was neutralized with 1 N HCl to a pH of 5 to 6. Extraction was conducted with ethyl acetate (3 × 20 mL). Resulting organic phases were combined and concentrated. The resulting crude product was purified by prep HPLC (ACCQPrep HP150, Welch Ultimate Polar-RP 5 µm 21.2 × 150 mm, (A) 0.1% TFA aq., and (B) CH₃CN). A purified product was collected and lyophilized to produce 0.28 g of *N*-(4-(2,4-dihydroxyphenyl)thiazol-2-yl)-2-thiomorpholin-2-oxoacetamide. ESI-MS: m/z388.1[M+Na]⁺; ¹H NMR(400MHz,DMSO-d₆):12.26(s,1H),10.21(s,1H),9.78(s,1H), 7.62(s,1H), 7.44(s,1H), 6.39(m,1H),6.28(m,1H),3.37(d,4H),2.68(d,4H).

A TYR inhibitory activity of a compound was assessed by a 3-methyl-2-benzothiazolinone hydrazine (MBTH) method modified with reference to the existing report (Journal of Biochemical and Biophysical Methods, 1994, 28 (3): 173-183). With MBTH as a chromogenic reagent, an inhibitory activity of a compound against human TYR was determined as follows: Levodopa was oxidized into L-dopaquinone by the human TYR. MBTH was then allowed to undergo a reaction with the L-dopaquinone to produce a colored product. The reaction was terminated, and the specific absorbance of the colored product at 505 nm was measured.

| Compound | Concentration | Inhibitory activity |
|---|---|---|
| *N*-(4-(2,4-dihydroxyphenyl)thiazol-2-yl)-2-oxopropanamide 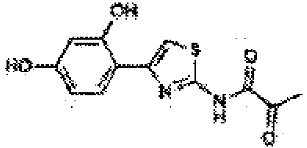 | 2 µg/mL | 92.6% |
| *N*-(4-(2,4-dihydroxyphenyl)thiazol-2-yl)-2-oxobutanamide 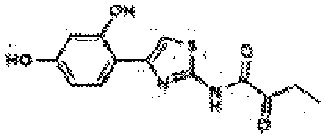 | 2 µg/mL | 90.0% |
| *N*-(4-(2,4-dihydroxyphenyl)thiazol-2-yl)-3-methyl-2-oxobutanamide 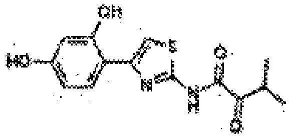 | 2 µg/mL | 78.5% |
| 2-cyclopropyl-*N*-(4-(2,4-dihydroxyphenyl)thiazol-2-yl)-2-oxoacetamide 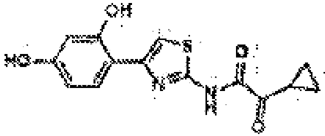 | 2 µg/mL | 75.3% |
| 2-cyclohexyl-N-(4-(2,4-dihydroxyphenyl)thiazol-2-yl)-2-oxoacetamide 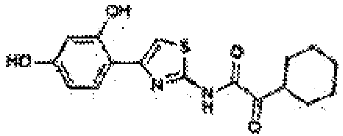 | 2 µg/mL | 65.0% |
| *N*-(4-(2,4-dihydroxyphenyl)thiazol-2-yl)-*N*',*N*'-dimethyloxalamide 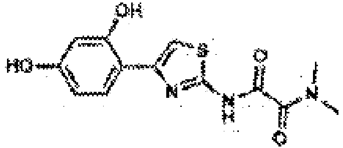 | 2 µg/mL | 90.7% |
| *N*-(4-(2,4-dihydroxyphenyl)thiazol-2-yl)-*N*',*N'-*diethyloxalamide 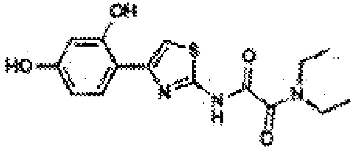 | 2 µg/mL | 89.2% |
| *N*-(4-(2,4-dihydroxyphenyl)thiazol-2-yl)-2-morpholino-2-oxoacetamide 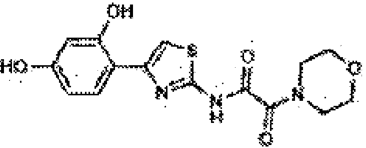 | 2 µg/mL | 86.1% |
| *N*-(4-(2,4-dihydroxyphenyl)thiazol-2-yl)-2-thiomorpholin-2-oxoacetamide 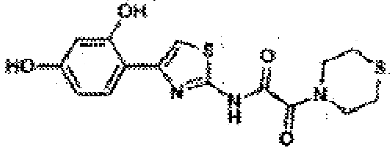 | 2 µg/mL | 76.5% |
| *N*-(4-(2,4-dihydroxyphenyl)thiazol-2-yl)-*N*'-isopropyloxalamide | 2 µg/mL | 96.7% |
| 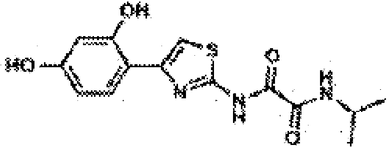 | | |
| *N*-(4-(2,4-Dihydroxyphenyl)thiazol-2-yl)-2-oxo-2-(pyrrolidin-1-yl)oxalamide 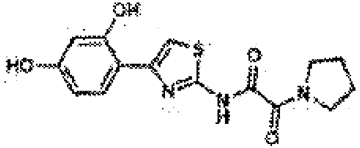 | 2 µg/mL | 93.5% |
| Methyl 2-((4-(2,4-dihydroxyphenyl)thiazol-2-yl)amino)-2-oxoacetate 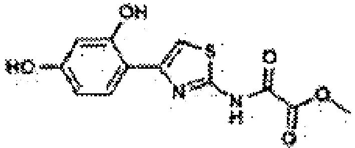 | 2 µg/mL | 88.1% |
| Ethyl 2-((4-(2,4-dihydroxyphenyl)thiazol-2-yl)amino)-2-oxoacetate 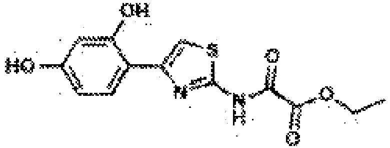 | 2 µg/mL | 89.6% |
| Cyclopropyl 2-((4-(2,4-dihydroxyphenyl)thiazol-2-yl)amino)-2-oxoacetate 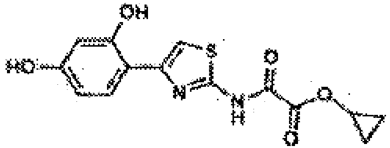 | 2 µg/mL | 85.8% |

### Use Examples

According to a mass fraction of each component in each formulation example provided in the table below, a desired emulsion could be prepared according to the following production process steps for a formulation example.

| Component | Content | | | |
|---|---|---|---|---|
| | Formulation 1 | Formulation 2 | Formulation 3 | Formulation 4 |
| Propylene glycol | 2.000 | 2.000 | 2.000 | 2.000 |
| Glycerin | 3.000 | 3.000 | 3.000 | 3.000 |
| Butylene glycol | 4.000 | 4.000 | 4.000 | 4.000 |
| Dipropylene glycol | 2.000 | 2.000 | 2.000 | 2.000 |
| Dimethicone | 2.000 | 2.000 | 2.000 | 2.000 |
| Cyclopentasiloxane | 2.000 | 2.000 | 2.000 | 2.000 |
| C₁₂-C₁₅ alkyl benzoate | 2.000 | 2.000 | 2.000 | 2.000 |
| Ethyl palmitate | 3.000 | 3.000 | 3.000 | 3.000 |
| Hydrogenated polydecene | 1.000 | 1.000 | 1.000 | 1.000 |
| Oleyl erucate | 0.200 | 0.200 | 0.200 | 0.200 |
| Polyglyceryl-3 methylglucose distearate | 1.500 | 1.500 | 1.500 | 1.500 |
| Carbomer | 0.100 | 0.100 | 0.100 | 0.100 |
| Ammonium acryloyldimethyltaurate/beheneth-25 methacrylate crosspolymer | 0.300 | 0.300 | 0.300 | 0.300 |
| Bis-ethoxydiglycol cyclohexane 1,4-dicarboxylate | 2.000 | 2.000 | 2.000 | 2.000 |
| Betaine salicylate | 1.000 | 1.000 | 1.000 | 1.000 |
| Glyceryl polymethacrylate | 2.000 | 2.000 | 2.000 | 2.000 |
| Aminomethyl propanol | 0.060 | 0.060 | 0.060 | 0.060 |
| Trisodium EDTA | 0.200 | 0.200 | 0.200 | 0.200 |
| Phenoxyethanol | 0.450 | 0.450 | 0.450 | 0.450 |
| Methylparaben | 0.200 | 0.200 | 0.200 | 0.200 |
| Butylated hydroxytoluene | 0.020 | 0.020 | 0.020 | 0.020 |
| N-(4-(2,4-dihydroxyphenyl)thiazol-2-yl)-2-oxopropanamide | 0.200 | 0.100 | - | 0.050 |
| *N*-(4-(2,4-dihydroxyphenyl)thiazol-2-yl)-*N'-*isopropyloxalamide | - | 0.200 | - | 0.100 |
| Ethyl 2-((4-(2,4-dihydroxyphenyl)thiazol-2-yl)amino)-2-oxoacetate | - | - | 0.300 | 0.200 |
| Fragrance | 0.080 | 0.080 | 0.080 | 0.080 |
| Water | The balance | The balance | The balance | The balance |

### Preparation process:

Formulation 1 was taken as an example.

Polyglyceryl-3 methylglucose distearate, hydrogenated polydecene, oleyl erucate, ethyl palmitate, dimethicone, methylparaben, and butylated hydroxytoluene were added to an oil-phase preparation tank, and heated until melted. Then, cyclopentasiloxane was added, and stirring was conducted for dispersion to produce an oil phase.

Water, glycerin, propylene glycol, butylene glycol, dipropylene glycol, betaine salicylate, trisodium EDTA, ammonium acryloyldimethyltaurate/beheneth-25 methacrylate crosspolymer, and carbomer were added to an aqueous-phase preparation tank, heated, and stirred until complete dissolution to produce an aqueous phase.

The aqueous phase was first transferred into an emulsification tank, and the oil phase was then added. Thorough stirring and homogenization were conducted. Stirring was conducted at a specified temperature.

Cooling was conducted. A mixture of glyceryl polymethacrylate, C₁₂-C₁₅ alkyl benzoate, and aminomethyl propanol was added, and thorough mixing was conducted. A mixture of *N*-(4-(2,4-dihydroxyphenyl)thiazol-2-yl)-2-oxopropanamide, fragrance, bis-ethoxydiglycol cyclohexane 1,4-dicarboxylate, and phenoxyethanol was added, and thorough homogenization was conducted. The resulting system was cooled to room temperature and allowed to stand to produce a desired emulsion.

The above examples are merely illustrative of some implementations of the present disclosure, and the description of these examples is specific and detailed, but should not be construed as a limitation to the scope of the present disclosure. It should be noted that those of ordinary skill in the art can further make several variations and improvements without departing from the concept of the present disclosure, and these variations and improvements all fall within the protection scope of the present disclosure. Therefore, the protection scope of the present disclosure should be subject to the protection scope defined by the claims.

## Claims

1. An α-ketoamide or substituted oxamide ester compound with a general structural formula shown in formula I: 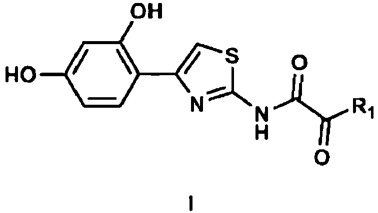
wherein R₁ is C₁-C₆ alkyl or cycloalkyl, or OR₂, wherein R₂ is C₁-C₈ alkyl or cycloalkyl, or NR₃R₄, wherein R₃ and R₄ are each independently C₁-C₆ alkyl or cycloalkyl; or
is an amine group with a following structure: 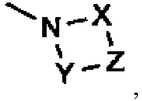 wherein X and Y are each independently C₁-C₅ methylene and Z is one of CH₂, O, S, and NH.

2. The α-ketoamide or substituted oxamide ester compound according to claim 1, wherein the α-ketoamide or substituted oxamide ester compound exists in a free form or as a pharmaceutically acceptable salt or ester thereof.

3. A composition comprising the α-ketoamide or substituted oxamide ester compound according to any one of claims 1 and 2.

4. The composition according to claim 3, wherein an addition amount of the α-ketoamide or substituted oxamide ester compound in the composition ranges from 0.000001% to 10% of a total weight of a preparation.
